# EUROPÄISCHE PATENTSCHRIFT

(11) **EP 0 835 643 B1**
(45) Veröffentlichungstag und Bekanntmachung des Hinweises auf die Patenterteilung: **27.08.2003**
(21) Anmeldenummer: 97117456.0
(22) Anmeldetag: 09.10.1997
(51) Int. Cl.: A61B 17/22, G01S 15/89

(54) **Schalltherapie**
Sonic therapy
Thérapie avec ultrasons

(30) Priorität: 11.10.1996 DE 19641935
(43) Veröffentlichungstag der Anmeldung: 15.04.1998
(73) Patentinhaber: Dornier MedTech Systems GmbH, 82234 Wessling (DE)
(72) Erfinder: Überle, Friedrich, Dipl.-Ing. Dr., 82205 Gilching (DE); Brandmeier, Richard, Dipl.-Ing., 86931 Prittriching (DE)
(74) Vertreter: Grünecker, Kinkeldey, Stockmair & Schwanhäusser Anwaltssozietät

(56) Entgegenhaltungen:
- EP-A- 0 355 175
- EP-A- 0 447 865
- EP-B- 0 301 360
- DE-A- 3 919 592
- DE-C- 19 543 344
- US-A- 5 329 928

## Beschreibung

Die vorliegende Erfindung betrifft ein Schalltherapiegerät zur berührungslosen Zertrümmerung von Konkrementen im Körper eines Patienten nach dem Oberbegriff des Anspruchs 1.

Aus der EP B 301360 ist ein Stoßwellengenerator zum berührungslosen Zertrümmern eines im Körper eines Lebewesen befindlichen Konkrementes mittels Stoßwellen bekannt, wobei er eine Stoßwellenquelle nach dem elektromagnetischen Prinzip aufweist, die einen Spulenträger, eine Flachspule und eine gegenüber der Flachspule isolierte metallische Membran umfaßt. Die Stoßwellenquelle ist über ein Koppelmedium auf das Konkrement ausrichtbar und mit einem Ultraschallkopf einer Ultraschall-Sende- und Empfangseinrichtung zum Senden eines akustischen Signals und zum Empfang von Echosignalen versehen, die durch Wechselwirkung des akustischen Sendesignals im Körper des Lebewesens entstehen zwecks Ortung und Beobachtung des Konkrements.

Eine exakte Beobachtung bei Verwendung einer elektromagnetischen Stoßwellenquelle des zu zertrümmernden Konkrements ist dabei dann möglich, wenn die Symmetrieachse der Abtastebene mit der Zentralachse der Stoßwellenquelle zusammenfällt. Im Spulenträger, in der Flachspule und in der metallischen Membran ist dabei jeweils eine zentrale Öffnung vorgesehen, in die der Ultraschallkopf der Ultraschall-Sende- und Empfangseinrichtung derart einführbar ist, daß er mit dem Koppelmedium in Berührung steht. Dadurch soll erreicht werden, daß die Ultraschallwellen und die Stoßwellen immer die gleiche Einfallrichtung aufweisen zur Gewährleistung der immer gleichen Zentrierung der beiden Systeme, um so die Steindesintegration kontinuierlich beobachten zu können. Die zentrische Anordnung des Ultraschallkopfes im Stoßwellengenerator soll für eine Symmetrierung der Stoßwellen sorgen. Das auftretende Maximum in der Mitte des Stoßwellengenerators wird reduziert.

Die EP A 355175 beschreibt eine Einrichtung zum berührungslosen Zertrümmern von Konkrementen im Körper eines Lebewesens mit einer Stoßwellenquelle zur Erzeugung von in einer Fokuszone zusammenlaufenden Stoßwellen mit einer Ultraschall-Ortungseinrichtung, die einen B-Scan-Applikator aufweist, mittels dessen zumindest die Fokuszone abtastbar ist und mit Mitteln zur akustischen Kopplung der Stoßwellenquelle und des B-Scan-Applikators mit dem Körper des Lebewesens, wobei der B-Scan-Applikator mehrere Ultraschall-Transducer enthält, mittels derer wenigstens quasigleichzeitig eine der Anzahl der Ultraschall-Transducer entsprechende Anzahl von nährungsweise parallel zueinander verlaufenden Schichten abtastbar ist, deren im Bereich der Fokuszone befindliche Abschnitte einander direkt benachbart sind. Damit wird die Möglichkeit geschaffen, Verlagerungen eines zu zertrümmernden Konkrementes quer zur Richtung der Schichten während der Behandlung zu beobachten, da das Konkrement bei Verlassen einer der Schichten in die jeweils direkt benachbarte Schicht eintritt und somit kontinuierlich abgebildet wird; dabei soll erkannt werden, in welcher Richtung sich das Konkrement verlagert, so daß die Lage der Einrichtung und des Körpers des zu behandelnden Lebewesens relativ zueinander korrigiert werden, bis sich das zu zertrümmernde Konkrement wieder in der Fokuszone, d. h. der mittleren B-Scan-Ebene befindet.

Diese bekannten Einrichtungen mit den bildgebenden Ultraschall-Scannern liefern also ein Real-Time-Bild des Behandlungsverlauf ohne zusätzliche Strahlenbelastung des Patienten und liefern damit dem Arzt eine einfache Orientierung. Jedoch sind die bekannten Einrichtungen noch mit dem Nachteil behaftet, daß die Scanner in die Stoßquelle mit eingebaut werden müssen (sogenannte Inline-Scanner), wobei dieser Einbau sehr aufwendig und kostenintensiv ist und eine speziell daran angepasste Modifikation der Schallquellen erfordert, die zu Energieverlusten bei der Therapie führen.

Aufgabe der vorliegenden Erfindung ist es, ein besonders einfaches Schalltherapiegerät zu schaffen, welches eine exakte Beobachtung und/oder Lokalisation des Konkrements mit einer beliebigen handelsüblichen Stoßwellenquelle ermöglicht.

Ausgehend von einem Schalltherapiegerät der eingangs näher genannten Art erfolgt die Lösung dieser Aufgabe mit dem im kennzeichnenden Teil des Anspruchs 1 angegebenen Merkmalen; vorteilhafte Ausgestaltungen sind in den Unteransprüchen beschrieben.

Die Erfindung bietet den Vorteil, daß durch das seitlichen Einführen des Scanners in den Koppelbalg (oder durch die Struktur der Stoßwellenquelle hindurch) eine optimale Adaption der Scanner-Parameter gewährleistet wird, daß der Scanner für die Ortung auf einfachste Art und Weise in seine jeweilige Position innerhalb des Koppelbalges gebracht werden kann, und daß bei Bedarf der Scanner aus dem Weg der Therapiewellen entfernt werden kann, um jegliche Abschattung des zu therapierenden Konkrementes zu vermeiden, bzw. um eine ungestörte Röntgenortung vornehmen zu können.

Zu diesem Zweck wird der Scanner (oder auch mehrere Scanner) durch die seitliche im Koppelbalg angeordneten Schleusen eingeführt, wobei die Halterung des eigentlichen Scanners entweder rechtwinklig zur Achse der Therapiewellen oder in einem Winkel dazu verlaufen kann. Diese Halterung nimmt auch die notwendigen Kabel für den Scanner auf.

Dabei ist es möglich, den Scanner entweder genau in die Achse der Therapiewellen einzuführen oder schräg dazu derart anzuordnen, daß der Fokusort der Therapiewellen in dem durch den Scanner erzeugten Ultraschallbild enthalten ist. Es ist ferner auch möglich, den Scanner oder die Scanner in den Koppelbalg entweder durch vollständiges Einbringen in die Koppelflüssigkeit anzuordnen oder aber den Scanner in einem vorzugsweise dünnwandigen, flexiblen, langgestreckten, dehnbaren Schlauch einzuführen, der sich in die Koppelflüssigkeit erstreckt. Zur besseren Kopplung zwischen Scanner und Koppelmedium kann der Scanner mit einem geeigneten Koppelgel oder einem Koppelöl bestrichen werden.

Um verschiedene Einbringtiefen zu unterstützen, können auch mehrere geeignete Durchführungen oder Schläuche im Koppelbalg angebracht werden.

Weiterhin ist es möglich, die Einlaßöffnungen, d. h. die Schleusen, in verschiedenen Positionen auf dem Koppelbalg zu verteilen, um so einen Zugang aus mehreren Richtungen zu erlauben. Besonders vorteilhaft ist es, wenn die Öffnungen jeweils um 90° zueinander versetzt sind, um dergestalt eine dreidimensionale Orientierung und Beobachtung des Konkrementes zu ermöglichen.

Im einfachsten Fall wird der Scanner mittels einer geeigneten Halterung an einem festen Ort eingebaut. Dabei kann er so justiert werden, daß die Bildachse genau mit der Achse der Therapiewellen übereinstimmt, so daß eine eindeutige Orientierung auch ohne die Einblendung der herkömmlichen Fadenkreuze ermöglicht wird, wenn, wie heutzutage üblich, im Ultraschallbild Entfernungsmarken eingeblendet werden und eine einblendbare Mittelachse zur Verfügung steht.

Bei einer weiteren Ausführungsform kann der Scanner mittels einer beweglichen Halterung derart bewegt werden, daß er bei der eigentlichen Therapie seitlich aus der Achse der Therapiewellen herausgeschwenkt wird und somit die Therapiewellen nur minimal abschattet, während er zum Orten und/oder Beobachten in eine axiale Position gebracht wird. Eine gezielte Verstellung außerhalb der Achse der Therapiewellen ist auch dann möglich, wenn z. B. bei der Nierenlithotripsie ein geeignetes Schallfenster zwischen den Rippen aufzusuchen ist.

Um den lateralen Blickwinkel des Scanners zu ändern, kann er entweder durch eines der versetzten Fenster eingebracht werden oder aber die Stoßwellenquelle kann um ihre Achse um einen Winkel, z. B. zwischen 90° und 110° verschwenkt werden. Eine weitere Möglichkeit zur genauen Positionsbestimmung des Scanners besteht in der Verwendung von Positionsaufnehmern an seiner Halterung, um dann im Ultraschallbild positionsrichtung eine Marke, z. B. ein Fadenkreuz zur Markierung der Fokusposition der Therapiewellen einzublenden. Gegebenenfalls kann auch der Scanner weitgehend frei beweglich sein, wobei in diesem Fall entweder ein magnetischer oder ein mit Ultraschall arbeitender Positionssensor an geeigneter Stelle der Scanner-Halterung angebracht wird, welcher für eine Einblendung einer Marke in den Therapiefokus im Ultraschallbild sorgt, z. B. mittels eines Video-Overlays. Dabei wird die Scanner-Halterung vorteilhafterweise derart ausgelegt, daß sie den Arzt bei der Suche des Zielortes möglichst wenig behindert, jedoch während der Therapie auf einfache Art und Weise arretiert werden kann.

Schließlich ist es möglich, durch geeignete motorische Schwenkung des Scanners um seine Achse eine dreidimensionale Darstellung des den Fokus einschließenden Volumens zu erzeugen, wodurch auch die Darstellung von senkrechten oder schrägen Schnitten ohne Veränderung der Scannerposition möglich ist.

Im folgenden wird die Erfindung anhand der Zeichnung näher erläutert, in der vorteilhafte Ausführungsbeispiele dargestellt sind. Es zeigen:
Figur 1 ein erstes Ausführungsbeispiel eines erfindungsgemäßen Schalltherapiegerätes,
Figur 2 die Anordnung von Positionsaufnehmern und/oder Verstellmotoren für die Halterung des Scanners,
Figur 3 ein weiteres Ausführungsbeispiel eines erfindungsgemäßen Schalltherapiegerätes,
Figur 4 eine Ansicht eines Koppelbalges mit mehreren Schleusen, und
Figur 5 einen Schnitt durch einen Koppelbalg mit verschiebbar gelagertem Scanner.

In den Figuren, in denen gleiche Teile mit gleichen Bezugszeichen versehen sind, ist schematisch ein Schalltherapiegerät zur berührungslosen Zertrümmerung von Konkremten im Körper eines Patienten dargestellt, wobei das Schalltherapiegerät eine Stoßwellenquelle 1 aufweist sowie ein Ultraschall-Ortungssystem in Form eines bildgebenden Scanners 3, der von einer Halterung 5 getragen wird, die gelenkig an einer Befestigung 4 angeordnet ist. Der Scanner 3 tastet den Fokusort F der von der Stoßwellenquelle 1 ausgesandten Therapiewellen ab, weil üblicherweise an diesem Fokusort das zu zertrümmernde Konkrement angeordnet ist.

Mit 2 ist ein üblicher Koppelbalg bezeichnet, in dem eine geeignete Koppelflüssigkeit zur Einleitung der Therapiewellen in den Körper des Patienten angeordnet ist.

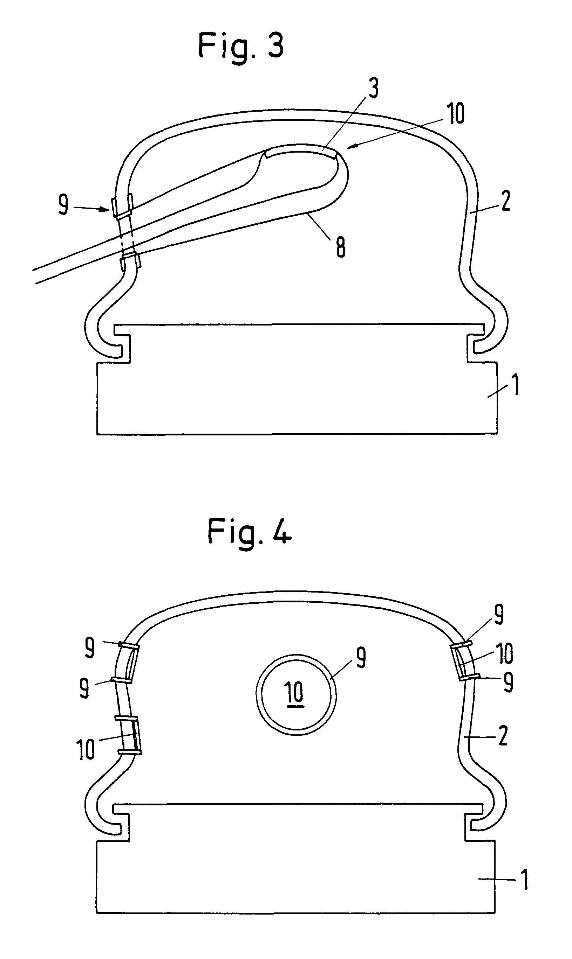

Erfindungsgemäß ist nun der Koppelbalg 2 mit mindestens einer seitlichen Schleuse 9 (Figur 4) versehen, die z. B. durch eine flexible dehnbare Membran 10 erschlossen ist zum Einführen des Scanners 3 in das Innere des Koppelbalgs 2, derart, daß die Halterung 5 des Scanners 2 einen Winkel mit der Achse der Therapiewellen einschließt.

Durch die seitliche Einführung des Scanners samt seiner Halterung durch eine der Öffnungen des Koppelbalgs hindurch entfällt jeglicher Eingriff in die Stoßwellenquelle selbst. Zusätzlich erzielt man noch den Vorteil des einfachen Auswechseln des Scanners zur optimalen Anpassung des Scannerparameter an die Therapie sowie des einfachen Einschubes des Scanners für die Behandlung bzw. Ortung bzw. Beobachtung durch geeignete Auswahl einer der Öffnungen des Koppelbalges. Bei Bedarf kann auch der Scanner vollständig aus dem Koppelbalg während der Zertrümmerung des Konkrementes entfernt werden, um eine Abschattung des zu therapierenden Gebietes im Körper zu minimieren oder auch für den Fall der Röntgenortung.

Als Scanner eignet sich z. B. ein Rektal- oder Gynäkologischer Scanner, wobei für oberflächennahe Ortungsaufgaben eine geeignete Schallfrequenz bei 7,5 MHz liegt, während für höhere Eindringtiefen, wie z. B. in der Gastroenterologie oder in der Nierenlithotripsie die 5 MHz bzw. 3,5 MHz-Scanner geeignet sind. Um eine räumliche Übersicht zu erhalten, können auch sogenannte "Biplane-Scanner" oder dreidimensionale Anordnungen eingesetzt werden.

Wie die Figuren und insbesondere Figur 4 erkennen lassen, wird der Scanner seitlich durch eine der Aussparungen im Koppelbalg in dessen Inneres eingeführt, wobei die Halterung entweder rechtwinklig zur Achse der Therapiewellen oder unter einem Winkel dazu verlaufen kann; in der Halterung sind auch vorteilhafterweise alle notwendigen Kabel für den Scanner enthalten. Der Scanner selbst kann entweder genau in der Achse der Therapiewellen angeordnet werden oder schräg dazu dergestalt, daß der Fokusort F der Therapiewellen im Ultraschallbildfenster enthalten ist.

Der Einbau des Scanners in das Koppelsystem kann entweder durch vollständiges Einbringen in die im Koppelbalg enthaltende Koppelflüssigkeit erfolgen oder aber der Scanner kann in einem vorzugsweise dünnwandigen flexiblen Schlauch 10 eingeführt werden, der sich dann mit dem Einschub des Scanners selbst bis in die Koppelflüssigkeit erstreckt. Zur besseren Kopplung zwischen Scanner und Koppelmedium kann der Scanner dabei mit einem geeigneten Koppelgel oder Koppelöl bestrichen werden. Um verschiedene Eindringtiefen zu unterstützen, können auch mehrere Schleusen, d. h. Durchführungen mit Membranen oder Schläuche im Koppelbalg angeordnet werden.

Weiterhin ist es möglich, wie insbesondere Figur 4 zeigt, die Schleusen 9 in verschiedenen Positionen auf dem Koppelbalg 2 zu verteilen, um so einen Zugang aus unterschiedlichen Richtungen zu ermöglichen. Besonders vorteilhaft ist es, wenn die Öffnungen um jeweils 90° zueinander versetzt sind, da dann eine einfache dreidimensionale Orientierung möglich ist.

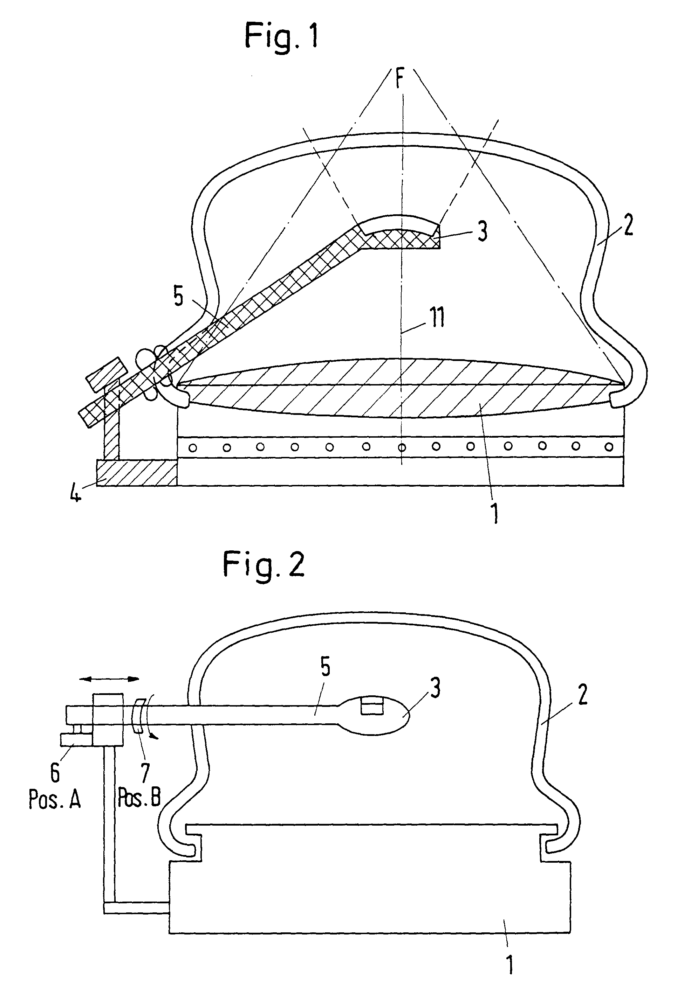

In Figur 2 sind schematisch mit 6 und 7 zwei Positionsgeber bezeichnet und durch den Doppelpfeil verschiedene Verstellmöglichkeiten des Scanners 3 innerhalb des Koppelbalges 2, wobei die Positionsgeber 6 und 7 dazu dienen, eine externe Ansteuerung des Scanners 3 durch die Halterung 5 hindurch vorzunehmen.

Figur 3 zeigt wie sich eine elastische Membran 10 in Form eines dünnen, langgestreckten Schlauches um den Scanner 3 herum erstreckt, nachdem dieser in das Innere des Koppelbalges 2 eingeführt worden ist.

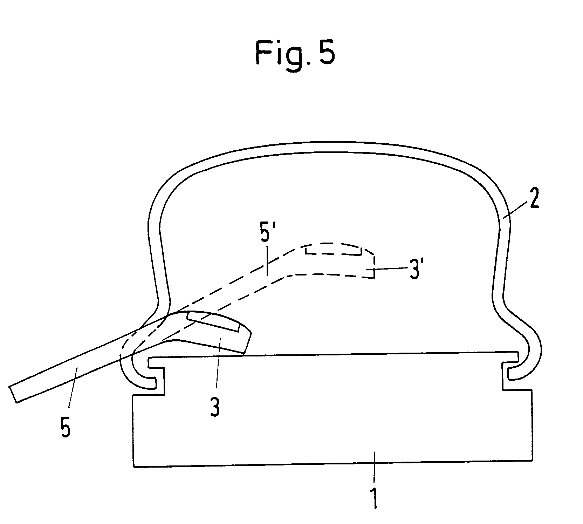

Figur 5 zeigt schließlich zwei unterschiedliche Anordnungen des Scanners 3, wobei dieser zur minimalen Abschattung während der Therapie sich, wie die ausgezogenen Linien zeigen, in der Nähe der Einführschleuse befindet und während seines Einsatzes, wie es gestrichelt dargestellt ist, in etwa der Achse der Therapiewelle. Eine gezielte Verstellung in eine Position außerhalb der Achse der Therapiewellen ist insbesondere bei der Nierenlithotripsie günstig, um ein geeignetes Schallfenster zwischen den Rippen aufzusuchen. Um den lateralen Blickwinkel des Scanners zu ändern, kann er entweder durch eine der versetzten Schleusen eingebracht werden, oder aber die Therapiequelle wird um die Achse der Therapiewellen in einem Winkel von 90 bis 110° verschwenkt.

Gegebenenfalls kann der Scanner auch weitgehend frei beweglich sein, wobei in diesem Fall z.B. ein magnetischer oder mit Ultraschall arbeitender Positionssensor (Figur 2) oder auch eine mechanische Verstellung der Scanner-Halterung erforderlich ist, die für eine Einblendung der Therapiefokusmarke im Ultraschallbild, z. B. mittels eines Video-Overlays sorgt. Die Halterung für den Scanner wird dabei vorteilhafterweise so ausgelegt, daß sie den Arzt bei der Suche des Zielorts möglichst wenig behindert, jedoch während der Therapie auf einfache Art arretiert werden kann.

## Patentansprüche

1. Schalltherapiegerät zur berührungslosen Zertrümmerung von Konkrementen im Körper eines Patienten, mit einer Stoßwellenquelle, mit einem Ultraschall-Ortungssystem in Form eines bildgebenden Scanners, der den Fokusort der von der Stoßwellenquelle ausgesandten Therapiewelle abtastet und mit einem Koppelbalg zwischen Stoßwellenquelle und Körperoberfläche des Patienten, **dadurch gekennzeichnet, daß** der Koppelbalg (2) oder eine Struktur der Stoßwellenquelle mit mindestens einer seitlichen Schleuse (9) versehen ist, die durch eine flexible, dehnbare Membran (10) verschlossen ist zum Einführen des Scanners (3) in das Innere des Koppelbalges, derart, daß die Halterung (5) des Scanners (3) einen Winkel mit der Achse (11) der Therapiewellen einschließt.

2. Schalltherapiegerät nach Anspruch 1, **dadurch gekennzeichnet, daß** der Scanner während seines Einsatzes derart im Koppelbalg angeordnet ist, daß der Therapiefokusort F in seinem erzeugten Bild enthalten ist.

3. Schalltherapiegerät nach einem der vorhergehenden Ansprüche, **dadurch gekennzeichnet, daß** die Schleuse durch eine Membran in Form von Segelklappen verschlossen ist.

4. Schalltherapiegerät nach einem der vorhergehenden Ansprüche, **dadurch gekennzeichnet, daß** der Scanner fest mit einer Halterung derart verbunden ist, daß er nach Ausrichtung auf den Therapiefokus über dem Ultraschallbild eingeblendete Marken den Therapiefokusort eindeutig identifiziert.

5. Schalltherapiegerät nach einem der vorhergehenden Ansprüche, **dadurch gekennzeichnet, daß** der Scanner beweglich an einer Halterung (4) derart angeordnet ist, daß eine eindeutige Zuordnung des Therapiefokus (F) zum Ultraschallbild ermöglicht wird.

6. Schalltherapiegerät nach einem der vorhergehenden Ansprüche, **dadurch gekennzeichnet, daß** der Scanner im Koppelbalg derart beweglich ist, daß er zum Orten und zur Therapie in unterschiedliche Positionen gebracht und dort festgehalten werden kann.

7. Schalltherapiegerät nach einem der vorhergehenden Ansprüche, **dadurch gekennzeichnet, daß** der Scanner mit einer Sensorik zur Ermittlung seines Ortes relativ zum Therapiefokus versehen ist, so daß der Scanner frei beweglich ist ohne die Anbindung des Fokus zu verlieren.

8. Schalltherapiegerät nach einem der vorhergehenden Ansprüche, **dadurch gekennzeichnet, daß** die Therapiequelle um die Stoßwellenachse verdrehbar und/oder verschwenkbar ist.

9. Schalltherapiegerät nach einem der vorhergehenden Ansprüche, **dadurch gekennzeichnet, daß** der Scanner mehrere Kristalle oder Kristallarrays aufweist oder mehrere mechanische Bewegungsachsen enthält, um simultan oder in Folge Bilder mehrerer Ebenen oder dreidimensionale Bilder aufzunehmen.

10. Schalltherapiegerät nach einem der vorhergehenden Ansprüche, **dadurch gekennzeichnet, daß** der Scanner an seiner dem Fokusort (F) abgewandten Rückseite durch einen mechanischen Schutz gegen die Einwirkung der Therapiewellen geschützt ist.

11. Schalltherapiegerät nach Anspruch 10, **dadurch gekennzeichnet, daß** der Schutz des Scanners aus einer Kombination aus geschlossenporigem Schaum und einer Metalloder Kunststoffplatte besteht.

12. Schalltherapiegerät nach einem der vorhergehenden Ansprüche, **dadurch gekennzeichnet, daß** es sich um einen mechanischen Scanner handelt.

13. Schalltherapiegerät nach einem der vorhergehenden Ansprüche, **dadurch gekennzeichnet, daß** der Scanner ein Linear-Scanner oder ein Curved-Array-Scanner ist.

14. Schalltherapiegerät nach einem der vorhergehenden Ansprüche, **dadurch gekennzeichnet, daß** die Scannerbefestigung mindestens einen Positionsgeber (6, 7) enthält, der extern angesteuert werden kann und der den Aufbau eines dreidimensionalen Ultraschallbildes mittels externer Bildverarbeitung ermöglicht.

15. Schalltherapiegerät nach einem der vorhergehenden Ansprüche, **dadurch gekennzeichnet, daß** der Scanner ein im wesentlichen rohrförmiges Gehäuse aufweist, daß die Scann-Ebene im wesentlichen senkrecht auf der Gehäuseachse steht und daß das rohrförmige Gehäuse im wesentlichen radial in die Therapiewellen einbringbar ist.

16. Schalltherapiegerät nach einem der vorhergehenden Ansprüche, **dadurch gekennzeichnet, daß** der bilderzeugende Ultraschall-Transducer des Scanners am Ende des rohrförmigen Gehäuses angeordnet ist.

## Claims

1. Sonic therapy device for contactless disintegration of concrements in a body of a patient, with a shock-wave source, with an ultrasonic positioning system in the form of an imaging scanner, which scans the focal position of the therapy wave emitted by the shock-wave source, and with a coupling bellow between the shock-wave source and the body surface of the patient, **characterised in that** the coupling bellow (2) or a structure of the shock-wave source is provided with at least one lateral port (9), which is closed by a flexible extensible diaphragm (10), for inserting the scanner (3) into the interior of the coupling bellow in such a way that the support (5) of the scanner (3) makes an angle with the axis (11) of the therapy waves.

2. Sonic therapy device according to Claim 1, **characterised in that**, during its use, the scanner is arranged in the coupling bellow in such a way that the therapy focal position F is contained in the image which it generates.

3. Sonic therapy device according to one of the preceding claims, **characterised in that** the port is closed by a diaphragm in the form of cuspid valves.

4. Sonic therapy device according to one of the preceding claims, **characterised in that** the scanner is firmly connected to a support in such a way that, after alignment with the therapy focus, it uniquely identifies the therapy focal position via marks superimposed on the ultrasonic image.

5. Sonic therapy device according to one of the preceding claims, **characterised in that** the scanner is arranged movably on a support (4) in such a way that unique assignment of the therapy focus (F) to the ultrasonic image is made possible.

6. Sonic therapy device according to one of the preceding claims, **characterised in that** the scanner is movable in the coupling bellow in such a way that it can be brought into different positions for positioning and for therapy, and held there.

7. Sonic therapy device according to one of the preceding claims, **characterised in that** the scanner is provided with a sensor system for determining its position relative to the therapy focus, so that the scanner is freely movable without losing the fix of the focus.

8. Sonic therapy device according to one of the preceding claims, **characterised in that** the therapy source can be rotated and/or tilted about the shock-wave axis.

9. Sonic therapy device according to one of the preceding claims, **characterised in that** the scanner has a plurality of crystals or crystal arrays, or contains a plurality of mechanical movement axes, in order to simultaneously or successively record images of a plurality of planes or three-dimensional images.

10. Sonic therapy device according to one of the preceding claims, **characterised in that** the scanner is protected against the effect of the therapy waves by mechanical protection at its rear side facing away from the focal position (F).

11. Sonic therapy device according to Claim 10, **characterised in that** the protection of the scanner consists of a combination of closed-pore foam and a metal or plastic plate.

12. Sonic therapy device according to one of the preceding claims, **characterised in that** a mechanical scanner is involved.

13. Sonic therapy device according to one of the preceding claims, **characterised in that** the scanner is a linear scanner or a curved-array scanner.

14. Sonic therapy device according to one of the preceding claims, **characterised in that** the scanner fixture contains at least one position encoder (6, 7), which can be externally driven and which makes it possible to construct a three-dimensional ultrasonic image by means of external image processing.

15. Sonic therapy device according to one of the preceding claims, **characterised in that** the scanner has an essentially tubular housing, **in that** the scan plane is substantially perpendicular to the housing axis, and **in that** the tubular housing can be brought substantially radially into the therapy waves.

16. Sonic therapy device according to one of the preceding claims, **characterised in that** the image-generating ultrasonic transducer of the scanner is arranged at the end of the tubular housing.

## Revendications

1. Appareil pour thérapie à ultrasons permettant de fragmenter, sans contact, des concrétions dans le corps d'un patient, comprenant une source d'ondes de choc, un système de localisation par ultrasons sous la forme d'un scanner fournissant des images qui balaye l'emplacement du foyer de l'onde thérapeutique émise par la source d'ondes de choc, et un coussin de couplage situé entre la source d'ondes de choc et la surface du corps du patient, **caractérisé en ce que** le coussin de couplage (2) ou une structure de la source d'ondes de choc est muni d'au moins un hublot (9) latéral qui est fermé par une membrane (10) flexible et extensible, afin de pouvoir introduire le scanner (3) à l'intérieur du coussin de couplage de sorte que le manche (5) du scanner (3) forme un angle avec l'axe (11) des ondes thérapeutiques.

2. Appareil pour thérapie à ultrasons selon la revendication 1, **caractérisé en ce que** le scanner est situé, pendant sa mise en oeuvre, dans le coussin de couplage de sorte que l'image produite comprend l'emplacement du foyer thérapeutique F.

3. Appareil pour thérapie à ultrasons selon l'une quelconque des revendications précédentes, **caractérisé en ce que** le hublot est fermé par une membrane en forme d'opercule à membrane extensible.

4. Appareil pour thérapie à ultrasons selon l'une quelconque des revendications précédentes, **caractérisé en ce que** le scanner est relié, de manière fixe, à un support de sorte qu'il détermine sans équivoque, après une orientation sur le foyer thérapeutique, l'emplacement du foyer thérapeutique, à l'aide des repères affichés sur l'image d'ultrasons.

5. Appareil pour thérapie à ultrasons selon l'une quelconque des revendications précédentes, **caractérisé en ce que** le scanner est disposé de manière mobile sur un support (4), afin de permettre une affectation sans équivoque du foyer thérapeutique (F) sur l'image d'ultrasons.

6. Appareil pour thérapie à ultrasons selon l'une quelconque des revendications précédentes, **caractérisé en ce que** le scanner est situé de manière mobile dans le coussin de couplage de sorte que l'on peut, afin de réaliser la localisation et la thérapie, le placer dans des positions différentes dans lesquelles on peut le bloquer.

7. Appareil pour thérapie à ultrasons selon l'une quelconque des revendications précédentes, **caractérisé en ce que** le scanner est équipé d'une technologie par capteurs permettant de déterminer sa position par rapport au foyer thérapeutique, de sorte que le scanner peut être déplacé librement perdre le contact avec le foyer.

8. Appareil pour thérapie à ultrasons selon l'une quelconque des revendications précédentes, **caractérisé en ce que** l'on peut faire tourner et/ou pivoter la source thérapeutique autour de l'axe des ondes de choc.

9. Appareil pour thérapie à ultrasons selon l'une quelconque des revendications précédentes, **caractérisé en ce que** le scanner présente plusieurs quartz ou matrices de quartz ou qu'il comprend plusieurs axes de mouvement mécaniques pour pouvoir enregistrer, simultanément ou successivement, des images sur plusieurs plans ou bien des images en trois dimensions.

10. Appareil pour thérapie à ultrasons selon l'une quelconque des revendications précédentes, **caractérisé en ce que** le scanner est protégé, sur son côté arrière qui est opposé à l'emplacement du foyer (F), par une protection mécanique, contre l'action des ondes thérapeutiques.

11. Appareil pour thérapie à ultrasons selon la revendication 10, **caractérisé en ce que** la protection du scanner est composée d'une combinaison d'une mousse à pores fermés et d'une plaque de métal ou de matière plastique.

12. Appareil pour thérapie à ultrasons selon l'une quelconque des revendications précédentes, **caractérisé en ce qu'**il s'agit d'un scanner mécanique.

13. Appareil pour thérapie à ultrasons selon l'une quelconque des revendications précédentes, **caractérisé en ce que** le scanner est un scanner à balayage linéaire ou un scanner à balayage courbe.

14. Appareil pour thérapie à ultrasons selon l'une quelconque des revendications précédentes, **caractérisé en ce que** la fixation du scanner comprend au moins un positionneur (6, 7) que l'on peut commander de manière externe et qui permet, au moyen d'un traitement externe des images, la production d'une image d'ultrasons en trois dimensions.

15. Appareil pour thérapie à ultrasons selon l'une quelconque des revendications précédentes, **caractérisé en ce que** le scanner présente un boîtier essentiellement tubulaire, que le plan de balayage est situé essentiellement perpendiculairement à l'axe du boîtier, et que l'on peut introduire, essentiellement dans le sens radial, le boîtier tubulaire dans les ondes thérapeutiques.

16. Appareil pour thérapie à ultrasons selon l'une quelconque des revendications précédentes, **caractérisé en ce que** le transducteur d'ultrasons produisant les images du scanner est disposé à l'extrémité du boîtier tubulaire.
